# EUROPEAN PATENT SPECIFICATION

(11) **EP 1 619 244 B1**
(45) Date of publication and mention of the grant of the patent: **09.06.2010**
(21) Application number: 05253870.9
(22) Date of filing: 22.06.2005
(51) Int. Cl.: C12N 5/074, A61K 38/18, A61K 31/7076, A61P 25/00

(54) **Use of stem cells for inducing neural differentiation**
Verwendung von Stammzellen zur Induktion der neuralen Differenzierung
Utilisation de cellules souche pour l'induction de la différentiation neurale

(30) Priority: 23.06.2004 US 873640
(43) Date of publication of application: 25.01.2006
(73) Proprietor: Taipei Veterans General Hospital, Beitou District, 112 Taipei City (TW)
(72) Inventor: Cheng, Henrich, Tapei Taiwan (CN); Tzeng, Shun-Fen, Tainan Taiwan (CN)
(74) Representative: Sexton, Jane Helen

(56) References cited:
- WO-A-03/018782
- WO-A-03/035852
- WO-A-03/092716
- WO-A-2004/020601
- US-A1- 2003 118 566
- TZENG SHUN-FEN ET AL: "Neuronal morphological change of size-sieved stem cells induced by neurotrophic stimuli" NEUROSCIENCE LETTERS, vol. 367, no. 1, 26 August 2004 (2004-08-26), pages 23-28, XP009054176 ISSN: 0304-3940
- OH Y S ET AL: "Differentiation of adult bone marrowstem cells into neuron-like cells" SOCIETY FOR NEUROSCIENCE ABSTRACTS, vol. 27, no. 1, 2001, page 58, XP009054180 & 31ST ANNUAL MEETING OF THE SOCIETY FOR NEUROSCIENCE; SAN DIEGO, CALIFORNIA, USA; NOVEMBER 10-15, 2001 ISSN: 0190-5295
- SANCHEZ-RAMOS J R: "NEURAL CELLS DERIVED FROM ADULT BONE MARROW AND UMBILICAL CORD BLOOD" JOURNAL OF NEUROSCIENCE RESEARCH, WILEY-LISS, US, vol. 69, no. 6, 15 September 2002 (2002-09-15), pages 880-893, XP008049059 ISSN: 0360-4012
- CHEN JIAN-RONG ET AL: "[Induced differentiation of rabbit bone marrow stromal cells into neuron-like cells]" DI YI JUN YI DA XUE XUE BAO = ACADEMIC JOURNAL OF THE FIRST MEDICAL COLLEGE OF PLA. APR 2004, vol. 24, no. 4, April 2004 (2004-04), pages 367-370, XP001207351 ISSN: 1000-2588
- YU K ET AL: "Adult rat bone marrow stromal cells were induced into neurons by neurotrophic factor" CHINESE OPHTHALMIC RESEARCH 2002 CHINA, vol. 20, no. 2, 2002, pages 97-100, XP001207352 ISSN: 1003-0808
- CHEN Z-Z ET AL: "Expression of Nestin in different stage of neural stem cells derived from rabbit bone marrow" CHINESE JOURNAL OF CLINICAL REHABILITATION 2004 CHINA, vol. 8, no. 7, 5 March 2004 (2004-03-05), pages 1243-1245, XP001207349 ISSN: 1671-5926
- HUNG SHIH-CHIEH ET AL: "In vitro differentiation of size-sieved stem cells into electrically active neural cells." STEM CELLS (DAYTON, OHIO) 2002, vol. 20, no. 6, 2002, pages 522-529, XP009054150 ISSN: 1066-5099
- STEPANOVA S B ET AL: "EFFECT OF ALKYLATING DERIVATIVES OF CYCLIC AMP ON THE PROLIFERATION OF MOUSE BONE MARROW STEM CELLS" BYULLETEN' EKSPERIMENTAL'NOI BIOLOGII I MEDITSINY, vol. 89, no. 5, 1980, pages 590-592, XP009054170 ISSN: 0365-9615

## Description

### BACKGROUND OF THE INVENTION

### 1. Field of the invention

The invention mainly relates to the treatment of neurological disorders.

### 2. Description of the Related Art

Loss of neural cells after injury of the central nervous system (CNS) makes CNS repair difficult. Many studies of show that neural stem cells (NSCs) isolated from various rodent and human CNS areas are capable of differentiating into neural cells in the adult rodent CNS under the influence of the environment and/or exogenous growth factors (F.H. Gage, Mammalian neural stem cells. Science. 287 (2000) 1433-1438; J. Price, B.P. Williams, Neural stem cells, Curr. Opin. Neurobiol. 11(2001) 564-567). Thus, replenishment of NSCs is thought to be a potential strategy for human CNS treatment (D.A. Peterson, Stem cells in brain plasticity and repair, Curr. Opin. Pharmacol. 2(2002) 34-42).

Stem cells derived from human bone marrow (hBMSCs) are heterogeneous in morphology. They multipotentially differentiate into osteoblasts, adipocytes, chondrocytes and muscle and can also generate neurons (E. Mezey, K.J. Chandross, G. Harta, R.A. Maki, S.R. McKercher, Turning Blood into Brain: Cells Bearing Neuronal Antigens Generated in vivo from Bone Marrow. Science, 290(2000) 1779-1782; E. Mezey, S. Key, G. Vogelsang, I. Szalayova, G.D. Lange, B. Crain, Transplanted bone marrow generates new neurons in human brains, Proc. Natl. Acad. Sci. U S A. 100(2003) 1364-1369; Sanchez-Ramos *et al*. (2000); D. Woodbury, E.J. Schwarz, D.J. Prockop, I.B. Black, Adult rat and human bone marrow stromal cells differentiate into neurons, J. Neurosci. Res. 61(2000) 364-370). Recently, human and mouse BMSCs have been reported to express neuronal progenitor marker (nestin), neuron-specific nuclear protein (NeuN), and glial acidic fibrillary protein (GFAP) after stimulation with retinoic acid and epidermal growth factor (EGF) or brain-derived neurotrophic factor (BDNF) (Sanchez-Ramos *et al*. (2000)). It has also been demonstrated that transplanted BMSCs are able to differentiate between the neuronal and glial lineages in damaged CNS (J.R. Sanchez-Ramos. Neural cells derived from adult bone marrow and umbilical cord blood. J. Neurosci Res. 69(2002) 880-893). Moreover, it is found in Chopp et al. that the transplantation of BMSCs can improve functional recovery in rats with focal cerebral ischemia (J. Chen, Y. Li, M. Chopp. Intracerebral transplantation of bone marrow with BDNF after MCAo in rat. Neuropharmacology. 39(2000) 711-716), in rats with traumatic brain injury (D. Lu, Y. Li, L. Wang, J. Chen, A. Mahmood, M. Chopp. Intraarterial administration of marrow stromal cells in a rat model of traumatic brain injury. J Neurotrauma. 18(2001) 813-819), and in mice with Parkinson's disease (Y. Li, J. Chen, L. Wang, L. Zhang, M. Lu, M. Chopp. Intracerebral transplantation of bone marrow stromal cells in a 1-methyl-4-phenyl-1,2,3,6-tetrahydropyridine mouse model of Parkinson's disease. Neurosci Lett. 316(2001) 67-70). These findings indicate the potential role as a useful cell source of CNS cell therapy in humans.

Nevertheless, the isolation of hBMSCs by adherence to the culture petri dish primarily generates heterogeneous populations (A.J. Friedenstein, J.E. Gorskaja, N.N. Kulagina. Fibroblast precursors in normal and irradiated mouse hematopoietic organs. Exp Hematol. 4(1976) 267-274). Therefore, several methods have been developed based on their different sizes and specific surface markers to generate homogeneous populations using cell sorting (S.C. Hung, N.J. Chen, S.L. Hsieh, H. Li, H.L. Ma, W.H. Lo, Isolation and characterization of size-sieved stem cells from human bone marrow, Stem Cells. 20 (2002) 249-258; R. Zohar, J. Sodek, C.A. McCulloch, Characterization of stromal progenitor cells enriched by flow cytometry, Blood. 90(1997) 3471-3481). Accordingly, *Hung et al.* (2002) have recently developed an efficient isolation of the homogeneous population from human bone marrow on the basis of cell size and adherent capacity via using Percoll gradient separation and a 3-µm porous sieve to dispose of smaller cells. The purified hBMSC population that was generated has been referred to as size-sieved cells (SSCs), and they have a greater renewal capability than heterogeneous populations of hBMSCs (Hung *et al.* (2002)). SSCs lack the surface markers of the early hematopoietic stem cells, CD34 and AC 133, at the passage 2 to 3, and fail to express markers for osteogenic MSCs and mature osteogenic precursors (Hung *et al.* (2002)). However, these cells express Thy-1, matrix receptors (CD44 and CD105), and integrins (CD29 and CD51). SSCs are multipotential, and can rise the osteogenic, adipogenic, and chondrogenic lineages under the influence of environmental signaling (Hung *et al.* (2002)). SSCs have also been found to generate neural cells electrically with the stimulation of antioxidant agents such as β-mercaptoethanol and retinoic acid, which are often used *in vitro* to induce the neural differentiation of stem cells (S.C. Hung, H. Cheng, C.Y. Pan, M.J. Tsai, L.S. Kao, H.L. Ma, In vitro differentiation of size-sieved stem cells into electrically active neural cells, Stem Cells. 20 (2002) 522-529). Although β-mercaptoethanol and retinoic acid have a potent effect on the differentiation of SSCs into functional neurons, the role of the two factors in CNS tissue repair remains to be defined.

However, stimulating SSCs to generate neural cells with antioxidant agents such as β-mercaptoethanol and retinoic acid can only be applied *in vitro*. β-Mercaptoethanol is a toxic reagent. Moreover, retinoic acid is a carcinogen. Both of the antioxidant agents cause damage to an animal and transplanting the stimulated neural cells leads to the receiver's death.

### SUMMARY OF THE INVENTION

The invention provides size-sieved stem cells derived from human bone marrow that have been treated with a) a neurotrophic factor comprising glial cell line-derived neurotrophic factor (GDNF) or b) a neurotrophic factor comprising pituitary adenylate cyclase-activating polypeptide (PACAP) for use in the treatment of neurological diseases. The invention also provides use of cells as defined above in the manufacture of a medicament for treating neurological disorders. Also described herein is a novel method for morphological transformation of SSCs from fibroblastic-like shapes to process-bearing forms with neurotrophic factors which is safe and effective in stimulating the changes of neural cell morphology. Furthermore, the neural cells obtained according to the method are suitable for repairing neural defects in animals.

Also described herein is a method for inducing neural differentiation comprising treating a bone marrow stem cell with a neurotrophic factor and/or dibutyryl cAMP (dbcAMP), wherein the neurotrophic factor comprises glial cell line-derived neurotrophic factor (GDNF) or pituitary adenylate cyclase-activating polypeptide (PACAP).

### BRIEF DESCRIPTION OF THE DRAWINGS

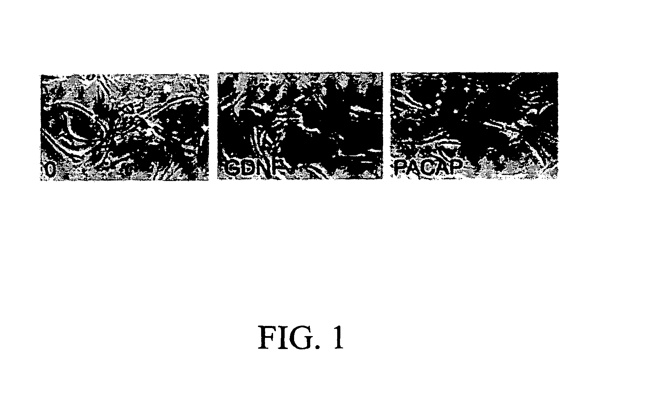

FIG. 1 illustrates phase contrast images of neuronal-like transformation of SSCs. It indicates that SSCs become process-bearing cells in ITS medium alone (0), and in ITS medium with 50 ng/ml GDNF or 20 ng/ml PACAP (magnification = 200 X).

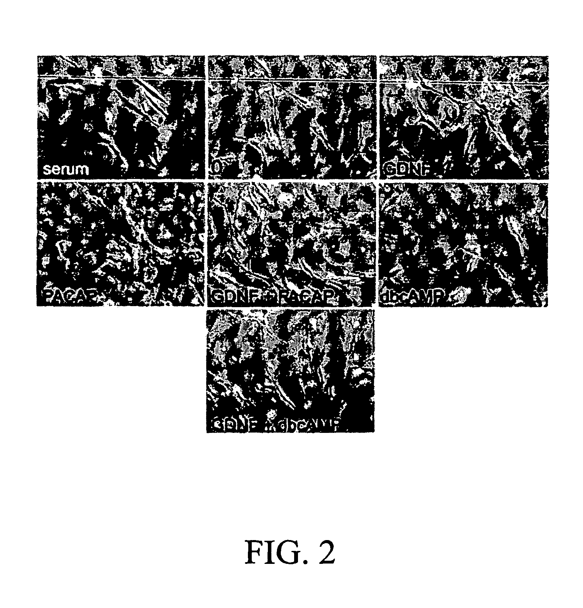

FIG. 2 illustrates phase contrast images of neuronal-like transformation of SSCs by GDNF, PACAP, and dbcAMP. It indicates that the morphology of SSCs in serum containing medium is fibroblastic-like, and becomes a process-bearing form in serum withdrawal medium (ITS medium alone; 0). Furthermore, the processes of SSCs treated for 7 days in ITS medium with GDNF (50 ng/ml), PACAP (20 ng/ml), dbcAMP (0.1 mM), GDNF (50 ng/ml) + PACAP (20 ng/ml), or GDNF (50 ng/ml) + dbcAMP (0.1 mM) are elongated and highly branching (magnification = 200 X).

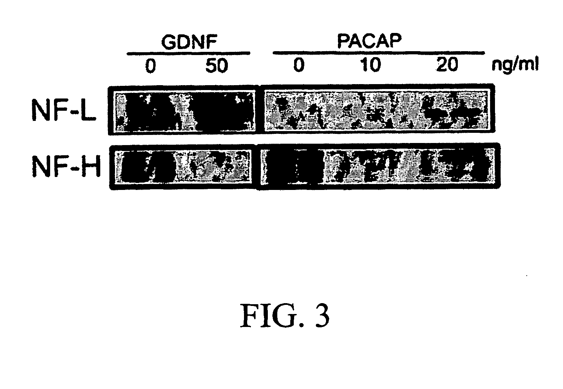

FIG. 3 illustrates the results of neuronal specific markers NF-L and NF-H expression wherein Western Blotting analysis showes that neuronal specific marker NF-L is upregulated in SSCs 7 days after treatment with GDNF (50ng/ml) or PACAP (10 and 20 ng/ml) in ITS medium when compared to that observed in ITS medium alone (0).

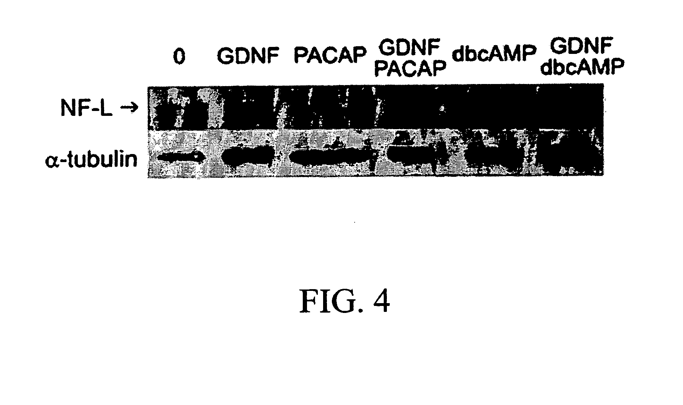

FIG. 4 illustrates the results of NF-L and α-tubulin expressions, wherein Western Blotting analysis shows that neuronal specific marker NF-L is upregulated in SSCs 7 days after treatments indicated as above. Moreover, the level of neuronal nonspecific cytoskeleton protein α-tubulin in SSCs is increased by various treatments indicated as above. Treatment with ITS-medium alone is represented as 0.

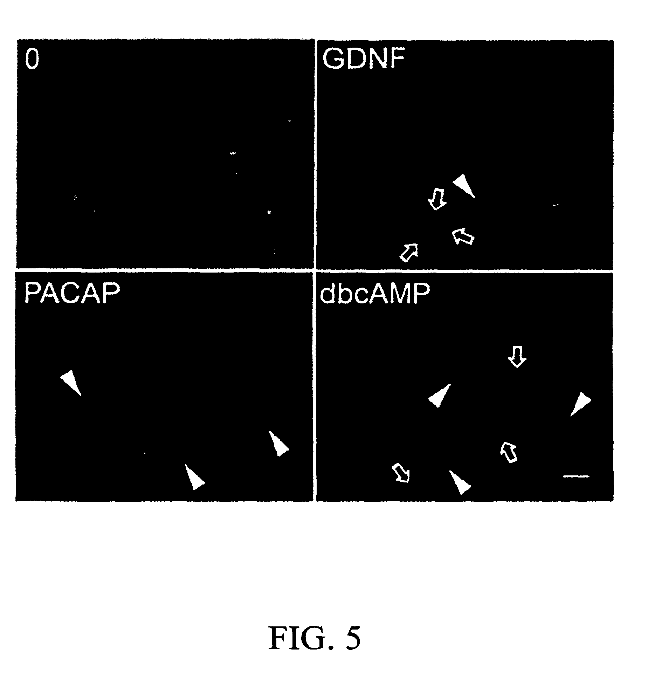

FIG. 5 illustrates the results of Immunofluorescence staining for α-intemexin in SSCs wherein SSCs are treated for 7 days in ITS medium alone (0) or with GDNF (50 ng/ml), PACAP (20 ng/ml), and dbcAMP (0.1 mM). The cultures are then subjected to immunofluorescence staining for α-intemexin. Branching (arrows) and elongation (arrowheads) of the processes in SSCs treated with GDNF and dbcAMP are more extensive over the ITS medium alone (0). Scale bar = 50 µm.

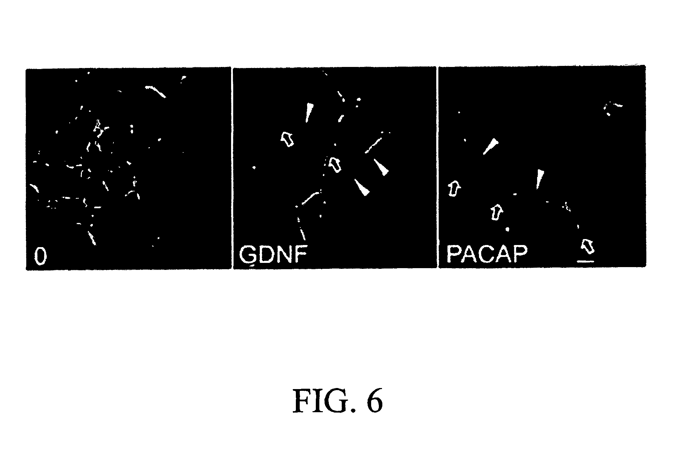

FIG. 6 illustrates images of neuronal-like transformation of SSCs. It indicate that the vesicle protein, synapsin-1, is already expressed when SSCs are cultured for 7 days in ITS medium alone (0). Moreover, treatment with GDNF (50 ng/ml) or PACAP (20 ng/ml) in ITS medium for 7 days induces further elongation (arrowheads) and increases branching of the processes (arrows). Scale bar = 50 µm.

### DETAILED DESCRIPTION OF THE INVENTION

The invention is size-sieved stem cells derived from human bone marrow that have been treated with a) a neurotrophic factor comprising glial cell line-derived neurotrophic factor (GDNF) or b) a neurotrophic factor comprising pituitary adenylate cyclase-activating polypeptide (PACAP) for use in the treatment of neurological diseases.

According to the invention, the bone marrow stem cell is taken for preparing functional neural cells. Stem cells derived from human bone marrow have the potential of differentiating into neurons and are considered to be the best material for regenerating neural cells. The bone marrow stem cell is a size-sieved stem cell derived from human bone marrow. Size-sieved stem cell is developed based on their different sizes and specific surface markers to generate homogeneous populations by using cell sorting to avoid heterogeneous population generation of primary bone marrow stem cells cultures. Furthermore, the size-sieved stem cells have greater renewal capability than heterogeneous populations. The size-sieved stem cell derived from human bone marrow is sieved more preferably with a 3-µm porous sieve. The size-sieved stem cells are efficiently isolated as the homogeneous population from human bone marrow on the basis of cell size and adherent capacity via using Percoll gradient separation and a porous sieve to dispose of smaller cells.

According to the invention, the neurotrophic factor is taken as a stimulus to induce neural differentiation. In view of the neurotrophic factor being a microenvironmental factor that exists in a normal physiological condition with less damage than that of a chemical reagent, it is regarded as a safe reagent to treat the bone marrow stem cell for the purpose of transplantation into an animal. According to the invention, the neurotrophic factor comprises glial cell line-derived neurotrophic factor or pituitary adenylate cyclase-activating polypeptide.

Glial cell line-derived neurotrophic factor (GDNF), which is a potent survival factor for many CNS neuronal cell populations, has the therapeutic potential for various CNS disorders. The therapeutic value of GDNF has been recently reviewed by Airaksinen and Saarma (M.S. Airaksinen, M. Saarma, The GDNF family: signalling, biological functions and therapeutic value. Nat Rev Neurosci. 3(2002) 383-394). It is also reported that intraspinal injection of GDNF into the injured spinal cord improved hindlimb recovery in rats with spinal cord injury (SCI) (H. Cheng, J.P. Wu, S.F. Tzeng, Neuroprotection of glial cell line-derived neurotrophic factor in damaged spinal cords following contusive injury. J. Neurosci. Res. 69 (2002) 397-405). According to the invention, treatment with GDNF improves the extension of neuritis and the extended processes with branching. In the aspect of neuron-specific markers, GDNF has a stimulatory effect on the expressions of neurofilament light protein (NF-L), vesicle protein-synapsin-1 and neuronal progenitor marker-internexin. GDNF also induces the neuronal nonspecific cytoskeleton protein α-tubulin expression in SSCs. Preferably, the dosage of glial cell line-derived neurotrophic factor is from 20 ng/mL to 50 ng/mL.

Pituitary adenylate cyclase-activating polypeptide (PACAP), a cAMP-inducing neuropeptide, has a critical role in CNS neural differentiation *in vivo* and *in vitro* (E. Dicicco-Bloom, N. Lu, J.E. Pintar, J. Zhang, The PACAP ligand/receptor system regulates cerebral cortical neurogenesis, Ann. N.Y. Acad. Sci. 11 (1998) 274-289; J.A. Waschek, Multiple actions of pituitary adenylyl cyclase activating peptide in nervous system development and regeneration, Dev. Neurosci. 24(2002)14-23). Moreover, elevated intracellular cAMP is decisive for axonal regeneration (W.D. Snider, F.Q. Zhou, J. Zhong, A. Markus, Signaling the pathway to regeneration, Neuron. 35(2002) 13-16). Gattei et al (V. Gattei, A. Celetti, A. Cerrato, M. Degan, A. De luliis, F.M. Rossi, G. Chiappetta, C. Consales, S. Improta, V. Zagonel, D. Aldinucci, V. Agosti, M. Santoro, G. Vecchio, A. Pinto, M. Grieco, Expression of the RET receptor tyrosine kinase and GDNFR-alpha in normal and leukemic human hematopoietic cells and stromal cells of the bone marrow microenvironment, Blood. 89 (1997) 2925-2937) have found that GDNF receptor-α and its accessory tyrosine kinase-RET were expressed in hBMSCs. According to the invention, PACAP is used to stimulate neuronal differentiation of the SSCs on the morphological transformation of the SSCs into neurons. PACAP stimulates neurogenesis via elevating intracellular cAMP through the PAC 1 receptor (Dicicco-Bloom *et al*. (1998)). According to the invention, treatment with PACAP improves the extension of neuritis and the extended processes with branching. In the aspect of neuron-specific markers, PACAP has a stimulatory effect on the expressions of NF-L, vesicle protein-synapsin-1 and neuronal progenitor marker-internexin. PACAP also induces α-tubulin expression in SSCs. Preferably, the dosage of pituitary adenylate cyclase-activating polypeptide is from 10 ng/mL to 20 ng/mL.

Dibutyryl cAMP, dbcAMP, is a cell permeable cAMP analog which induces highly branched, elongated, and delicate processes in SSCs. According to the invention, treatment with dbcAMP increases the expressions of NF-L, resicle protein-synapsin-1 and neuronal progenitor marker-internexin. dbcAMP also induces α-tubulin expression in SSCs. Furthermore, treatment with dbcAMP caused more extensive branched, elongated processes than those observed in GDNF- and PACAP-treated SSC cultures. Preferably, the dosage of dibutyryl cAMP is 100 µM.

Neurotrophic factors have effect on neuronal survival and repair for many neurological diseases. Thus, the combination of cell transplantation with neurotrophic factors is thought to be a potent therapeutic strategy for neurological diseases. It is evidenced neural stem cell differentiation is induced by neurotrophic factors (N.Y. Ip, The neurotrophins and neuropoietic cytokines: two families of growth factors actingon neural and hematopoietic cells, Ann. N.Y. Acad. Sci. 840 (1998) 97-106; A. Markus, T.D. Patel, W.D. Snider, Neurotrophic factors and axonal growth, Curr. Opin. Neurobiol.12(2002) 523-531; H. Thoenen, Neurotrophins and neuronal plasticity, Science. 270(1995) 593-598). However, the effect of neurotrophic factors on trans-differentiation of SSCs into neuronal cells is unknown. The invention is the first to show that GDNF and PACAP can stimulate SSC differentiation toward a mature neuronal phenotype. The two neurotrophic factors are known to exert neuroprotection and to stimulate axonal regrowth via activating cAMP/PKA, MAP kinase, PI3 kinase, and PLC-γ signaling pathways (Airaksinen *et al.* (2002) and Waschek *et al.* (2002)). In addition, elevating intracellular cAMP can enhance the formation of neurites in SSCs. The present invention showed that SSCs cultured in ITS medium have a process-bearing form and are positive for neuronal synapsin vesicle protein-synapsin-1. Treatment with GDNF or PACAP in ITS medium can further induce the transformation of SSCs into neuronal-like cells with dedicated processes. NF proteins expressed in most CNS mature neurons are known to play a crucial role in neuronal growth, organization, shape, and plasticity. Therefore, the additive expression of NF-L in SSCs induced by ITS medium containing GDNF or PACAP indicates the regulatory role of the two molecules on neuronal differentiation of SSCs. Extensive branching in elongated processes and increased levels of NF-L are observed in dbcAMP-treated SSCs, suggesting that intracellular cAMP may be involved in promoting neuronal differentiation of PACAP-treated SSCs. Note that no synergetic effect of GDNF combines with either PACAP or dbcAMP in the production of NF-L and α-tubulin in SSCs.

The following Examples are given for the purpose of illustration only and are not intended to limit the scope of the present invention.

### Example 1: Size-Sieved Stem Cell Derived from Human Bone Marrow

SSCs were isolated from human bone marrow as described previously (Hung *et al*. (2002)). In brief, human bone marrow was aspirated from the iliac crest of normal donors, and then washed twice with phosphate-buffered saline (PBS). The cells were loaded into 1.073 g/ml Percoll solution (Sigma®), and then centrifuged at 900 g for 30 min. The mononuclear cells (MNCs) were collected from the interface, and plated at the density of 1 x 10⁶ MNCs/cm² onto a 10-cm plastic culture dish comprised of an inserted sieve with 3-µm pores (Transwell System, Corning®). The cells were cultured in Dulbecco's modified Eagle's medium-low glucose (DMFM-LG) containing 10 % fetal bovine serum (FBS), 100 U/ml penicillin, 100 mg/ml streptomycin, and 0.25 µg/ml amphotericin B (serum-containing medium). After 7 days, the cells adhering to the upper part of the inserted sieve had a larger, fibroblastic-like morphology, and were named SSCs. However, the cells that passed through the sieve were small, polygonal and had less renewal. SSCs were then harvested with 0.25 % trypsin and 1 mM EDTA, and replated on 10-cm culture petri dishes. When SSCs were grown in serum containing medium to 80 % confluence, the cells were replated onto 35-mm culture petri dishes at the density of 1 x 10⁵ cells/dish for Western Blotting, or onto 8-well chambers at the density of 1 x 10⁴ cells/well for immunofluorescence.

### Example 2: Stimulation of Size-Sieved Stem Cell Derived from Human Bone Marrow

After 48 h in serum containing medium, SSCs were treated in serum medium (ITS medium) alone, and in ITS medium containing GDNF (20 and 50 ng/ml, R&D ®), PACAP (10 and 20 ng/ml; Sigma®), or dbcAMP (20 µM; Sigma®). ITS medium consisted of 56 % DMEM-LG (Life Biotech®), 40 % MCDB-201 medium (Sigma®), and 1X ITS medium supplement (Sigma®) contained 1 mg/ml insulin, 0.55 mg/ml human transferrin, 0.5 µg/ml sodium selenite, 10 nM dexamethasone (Sigma®), and 10 µM ascorbic acid (Sigma®). We found that SSCs in DMEM-LG medium without serum were founded to respond poorly to GDNF and PACAP. However, when SSCs were cultured in serum free medium with ITS supplement, these cells well adhered onto cultured plates and extended their processes. Therefore, treatments were performed in ITS medium.

The morphology of SSCs is as shown in FIGs. 1 and 2. As shown in FIG. 2, when SSCs were cultured in 10 % FBS-containing medium, the cells had a flat, fibroblastic-like morphology. However, SSCs were found to generate extended neurites while SSCs were cultured in ITS medium alone (FIGs. 1 and 2 ). In addition, treatment with SSCs in ITS medium containing GDNF or PACAP further improved the extension of neuritis. It indicated that the vesicle protein, synapsin-1, was already observed when SSCs were cultured for 7 days in ITS medium alone. Given the above, ITS medium alone also induced the neuronal differentiation of SSCs to some extent.

### Example 3: Effects of GDNF and PACAP and dbcAMP on Neuron-Specific Markers

This is to study the effects of GDNF, PACAP and dbcAMP on neuronal differentiation of SSC in ITS medium. The levels of neuron-specific markers (NF-L and NF-H) in the SSCs were examined by Western Blotting. The cells were replated at the density of 1 x 10⁵ cells per 35 mm petri dish and cultured for 7 days in ITS medium with GDNF, PACAP or dbcAMP at the distinct concentrations. Cells were harvested and gently homogenized on ice using PBS containing 1% SDS, 1 mM phenylmethyl-sulfonylfluoride (PMSF), 1 mM EDTA, 1.5 mM pepstatin, 2 mM leupeptin, and 0.7 mM aprotinin. Protein concentrations were determined using a Bio-Rad® DC kit. Ten µg of total protein was loaded onto 7.5 % SDS-PAGE, and transferred to a nitrocellulous membrane. NF-L (70 kDa) and NF-H (200 kDa) were identified by incubating the membrane with anti-NF antibodies (Chemicon®) overnight at 4 °C, followed by horseradish peroxidase-conjugated secondary antibodies and enhanced chemiluminescence solution (NEN LifeScience®).

The results are shown in FIGs. 3 and 4. Referring to FIG. 3, Western Blotting showed that treatment with GDNF or PACAP had a stimulatory effect on the expression of NF-L protein in SSCs, albeit less effect on NF-H levels in SSCs.

As shown in FIG. 4, ITS medium containing the cell permeable cAMP analog, dbcAMP, induced highly branched, elongated, and delicate processes in SSCs when compared to those observed in ITS medium alone. As with GDNF and PCACP, Western Blotting also indicated that treatment with dbcAMP increased the level of NF-L and α-tubulin in SSCs. Note that there was no synergetic effect of GDNF combined with either PACAP or dbcAMP on the production of NF-L and α-tubulin in SSCs.

Immunofluorescence staining for another neuronal intermediate filament protein, α-internexin (FIG. 5), was performed using antibodies against α-internexin (1:200; Chemicon®). It was evidenced that GDNF and PACAP could induce process branching of SSCs to some extent. However, treatment with dbcAMP for 7 days caused more extensive branched, elongated processes than those observed in GDNF- and PACAP-treated SSC cultures, as well as control cultures.

### Example 4: Effects of GDNF and PACAP on Morphological Change of SSCs

To detect the morphological change of the SSCs that had been treated with 50 ng/ml GDNF or 20 nM PACAP in ITS medium for 7 days, the SSCs were fixed in PBS-containing 4 % paraformaldehyde for 10 minutes. Examination of immunofluorescence for synapsin-1, a synapse vesicle protein, was performed by incubating SSCs with anti-synapsin-1 antibodies (1:200; BD Biosciences®) in PBS-containing 0.1 % Triton X-100 and 5 % horse serum overnight at 4 °C, followed by biotinylated secondary antibodies (1:200; Vector®) and FITC-avidin (1:200; Vector®). The results indicated that a strong immunofluorescence staining for synapsin-1 was observed in the processes and/or peripheral membranes of SSCs with or without treatment. Yet, it was evident that synapsin-1 positive cells in GDNF- or PACAP-treated cultures showed extended processes with branching (as shown in FIG. 6).

## Claims

1. Size-sieved stem cells derived from human bone marrow that have been treated with
a) a neurotrophic factor comprising glial cell line-derived neurotrophic factor (GDNF) or
b) a neurotrophic factor comprising pituitary adenylate cyclase-activating polypeptide (PACAP)
for use in the treatment of neurological diseases.

2. Cells for use according to Claim 1, wherein the size-sieved stem cells derived from human bone marrow are sieved with a 3-µm porous sieve.

3. Cells for use according to Claim 1 or 2, wherein the neurotrophic factor comprises glial cell line-derived neurotrophic factor.

4. Cells for use according to Claim 3, wherein the dosage of glial cell line-derived neurotrophic factor is from 20 ng/mL to 50 ng/mL.

5. Cells for use according to Claim 1 or 2, wherein the neurotrophic factor comprises pituitary adenylate cyclase-activating polypeptide.

6. Cells for use according to Claim 5, wherein the dosage of pituitary adenylate cyclase-activating polypeptide is from 10 ng/mL to 20 ng/mL.

7. Cells for use according to any one of Claims 1 to 6, wherein the neurotrophic factor induces neural differentiation of the size-sieved stem cells derived from human bone marrow.

8. Cells for use according to Claim 7, wherein the neural differentiation comprises neurofilament light protein (NF-L) increasing, α-tubulin increasing, vesicle protein-synapsin-1 production, neuronal progenitor marker-internexin production, cell processes elongation, and process branching increasing.

9. Use of cells as defined in any one of Claims 1 to 8 in the manufacture of a medicament for treating neurological disorders.

## Patentansprüche

1. Größengesiebte, aus menschlichem Knochenmark abgeleitete Stammzellen, die behandelt wurden mit
a) einem neurotrophischen Faktor, der von Gliazelllinie abgeleiteten neurotrophischen Faktor (GDNF) enthält, oder
b) einem neurotrophischen Faktor, der hypophysäre Adenylatcyclase aktivierendes Polypeptid (PACAP) enthält,
zur Verwendung bei der Behandlung neurologischer Krankheiten.

2. Zellen zur Verwendung nach Anspruch 1, wobei die größengesiebten, aus menschlichem Knochenmark abgeleiteten Stammzellen mit einem 3-µm porösen Sieb gesiebt sind.

3. Zellen zur Verwendung nach Anspruch 1 oder 2, wobei der neurotrophische Faktor von Gliazelllinie abgeleiteten neurotrophischen Faktor enthält.

4. Zellen zur Verwendung nach Anspruch 3, wobei die Dosierung des von Gliazelllinie abgeleiteten neurotrophischen Faktors von 20 ng/mL bis 50 ng/mL beträgt.

5. Zellen zur Verwendung nach Anspruch 1 oder 2, wobei der neurotrophische Faktor hypophysäre Adenylatcyclase aktivierendes Polypeptid enthält.

6. Zellen zur Verwendung nach Anspruch 5, wobei die Dosierung des hypophysäre Adenylatcyclase aktivierenden Polypeptids von 10 ng/mL bis 20 ng/mL beträgt.

7. Zellen zur Verwendung nach einem der Ansprüche 1 bis 6, wobei der neurotrophische Faktor die neurale Differenzierung der größengesiebten, aus menschlichem Knochenmark abgeleiteten Stammzellen induziert.

8. Zellen zur Verwendung nach Anspruch 7, wobei die neurale Differenzierung die Zunahme von Neurofilament-Light-Protein (NF-L), die Zunahme von α-Tubulin, die Produktion von Vesikelprotein-Synapsin-1, die Produktion von neuronalem Progenitormarker-Internexin, die Verlängerung von Zellprozessen und die Zunahme von Prozessverzweigung umfasst.

9. Verwendung von Zellen wie in einem der Ansprüche 1 bis 8 definiert zur Herstellung eines Arzneimittels zur Behandlung neurologischer Störungen.

## Revendications

1. Cellules souches dérivées de moelle osseuse humaine, triées selon leur taille, qui ont été traitées avec :
a) un facteur neurotrophique comprenant du facteur GDNF (facteur neurotrophique dérivé de cellules gliales),
b) ou un facteur neurotrophique comprenant du polypeptide PACAP (polypeptide activateur de l'adényl-cyclase hypophysaire),
pour utilisation dans le traitement de troubles neurologiques.

2. Cellules pour utilisation, conformes à la revendication 1, lesquelles cellules souches dérivées de moelle osseuse humaine et triées selon leur taille ont été triées au moyen d'une membrane poreuse de 3 µm de taille de pores.

3. Cellules pour utilisation, conformes à la revendication 1 ou 2, pour lesquelles le facteur neurotrophique comprenait du facteur GDNF (facteur neurotrophique dérivé de cellules gliales).

4. Cellules pour utilisation, conformes à la revendication 3, pour lesquelles la concentration du facteur GDNF valait de 20 à 50 ng/mL.

5. Cellules pour utilisation, conformes à la revendication 1 ou 2, pour lesquelles le facteur neurotrophique comprenait du polypeptide PACAP (polypeptide activateur de l'adényl-cyclase hypophysaire).

6. Cellules pour utilisation, conformes à la revendication 5, pour lesquelles la concentration du polypeptide PACAP valait de 10 à 20 ng/mL.

7. Cellules pour utilisation, conformes à l'une des revendications 1 à 6, chez lesquelles le facteur neurotrophique induit une différentiation neurale des cellules souches dérivées de moelle osseuse humaine et triées selon leur taille.

8. Cellules pour utilisation, conformes à la revendication 7, chez lesquelles ladite différentiation neurale comprend une augmentation du taux de protéine NF-L (protéine de chaîne légère de neurofilament), une augmentation du taux d'alpha-tubuline, la production de protéine vésiculaire synapsine 1, la production de marqueur de cellules progénitrices neuronales internexine, un allongement des prolongements cellulaires, ou une augmentation du degré de ramification des prolongements cellulaires.

9. Utilisation de cellules conformes à l'une des revendications 1 à 8 dans la fabrication d'un médicament conçu pour le traitement de troubles neurologiques.
